# EUROPÄISCHE PATENTANMELDUNG

(11) **EP 4 821 448 A2**
(43) Veröffentlichungstag der Anmeldung: **14.10.2026**
(21) Anmeldenummer: 26153361.6
(22) Anmeldetag: 22.01.2026
(51) Int. Cl.: A61B 17/17, A61B 17/74, A61B 17/88, A61B 17/16

(54) **L-FÖRMIGES WERKZEUG FÜR EIN SET ZUR VERSORGUNG VON KNOCHENBRÜCHEN**

(30) Priorität: 20.02.2025 AT 501202025
(71) Anmelder: I.T.S. GmbH, 8301 Lassnitzhöhe (AT)
(72) Erfinder: Auf, Wolfgang, 8075 Hart bei Graz (AT); Resedaritz, Thomas, 8264 Großhartmannsdorf (AT); Prager, Ronald, 24796 Bovenau (DE)
(74) Vertreter: Wirnsberger & Lerchbaum Patentanwälte OG

(57) **Zusammenfassung**

Die Erfindung betrifft ein Werkzeug (6) für ein Set zur Versorgung von Knochenbrüchen, insbesondere proximalen Femurfrakturen, umfassend einen Grundkörper (601) mit einem ersten, insbesondere proximalen, Ende (602) und einem zweiten, insbesondere distalen, Ende (603) und einem Greifbereich (604) sowie einen Verbindungsbereich (605) zum Anlegen des Werkzeuges (6) an einen Teil des Sets, insbesondere eine Knochenschraube (3). Um bei einem Set zur Versorgung von insbesondere proximalen Femurfrakturen mit einer Knochenschraube (3) sowie einer mit dieser zusammenwirkenden Stellschraube (4) eine genaue Ausrichtung der Knochenschraube (3) beim bzw. vor dem Einführen der Stellschraube (4) zu ermöglichen, weist der Verbindungsbereich (605) eine Nut (606) auf.

Des Weiteren umfasst die Erfindung ein Set zur Versorgung von Knochenbrüchen, insbesondere proximalen Femurfrakturen sowie ein Verfahren zur Versorgung von Knochenbrüchen insbesondere mit einem derartigen Set.

## Beschreibung

Die Erfindung betrifft ein Werkzeug für ein Set zur Versorgung von Knochenbrüchen, insbesondere proximalen Femurfrakturen, umfassend einen Grundkörper mit einem ersten, insbesondere proximalen, Ende und einem zweiten, insbesondere distalen, Ende und einem Greifbereich und einem Verbindungsbereich zum Anlegen des Werkzeuges an einen Teil des Sets, insbesondere eine Knochenschraube.

Des Weiteren betrifft die Erfindung ein Set zur Versorgung von Knochenbrüchen, insbesondere proximalen Femurfrakturen, umfassend einen Knochennagel, eine Knochenschraube und eine Stellschraube, wobei der Knochennagel eine Öffnung aufweist, durch welche die Knochenschraube geführt ist, und wobei die Stellschraube eingerichtet ist, mit der Knochenschraube in Kontakt gebracht zu werden, um zumindest eine Bewegungsmöglichkeit der Knochenschraube festzulegen.

Des Weiteren betrifft die Erfindung ein Verfahren zur Versorgung von Knochenbrüchen, insbesondere proximalen Femurfrakturen, insbesondere mit einem Set der eingangs genannten Art und/oder unter Einsatz eines Werkzeuges der vorstehenden genannten Art, wobei eine Knochenschraube und eine mit der Knochenschraube zusammenwirkende Stellschraube durch eine Öffnung eines in einem Knochenteil angeordneten Knochennagels geführt werden, sodass die Stellschraube schräg zur Knochenschraube verläuft, wobei die Stellschraube eingerichtet ist, mit der Knochenschraube in Kontakt gebracht zu werden, um zumindest eine Bewegungsmöglichkeit der Knochenschraube festzulegen.

Femurfrakturen insbesondere im proximalen Bereich bedürfen einer sorgfältigen Versorgung. Derartige Frakturen wie Oberschenkelhalsbrüche treten vor allem bei älteren Menschen auf. Da sich eine Knochensubstanz bei älteren Menschen häufig nicht mehr in einem Idealzustand befindet, können sich Operationen zur Versorgung von Femurfrakturen insbesondere im proximalen Bereich als anspruchsvoll darstellen. Umso wichtiger ist es, einen Operationsverlauf für einen Operateur möglichst einfach, aber auch mit ausreichenden Möglichkeiten zur individuellen Anpassung des für die Versorgung eingebrachten Implantats zu ermöglichen. Unvorhergesehene Komplikationen beim Einbringen des Implantats können dazu führen, dass bei der schrittweisen Abfolge des Einbringens des Implantats einzelne Schritte wieder rückgängig und von Neuem gestartet werden müssen, was nicht nur eine Operationsdauer verlängert, sondern auch zu einer psychischen Belastung des Operateurs führt und darüber hinaus selbstredend aufgrund zusätzlicher Verfahrensschritte die Gefahr von Komplikationen erhöht. Insofern liegt ein gewisser Zielkonflikt vor, weil Instrumente zum Einbringen von Implantaten zur Versorgung von insbesondere proximalen Femurfrakturen auf der einen Seite möglichst einfach aufgebaut sein sollen, um Komplikationen beim Setzen des Implantats zu vermeiden, auf der anderen Seite aber trotz der einfachen Bedienbarkeit größtmögliche Flexibilität während der Operation bieten sollen.

Aus dem Stand der Technik sind diverse Sets zur Versorgung von insbesondere proximalen Femurfrakturen bekannt geworden. Heute gelten Sets als technologisch führend, welche bei der Versorgung von proximalen Femurfrakturen neben einem einzubringenden Knochennagel, der in den Oberschenkelhals eingeführt wird, eine Knochenschraube sowie eine weitere Schraube zur Festlegung der Knochenschraube umfassen. Bei diesem zumindest dreiteiligen Set weist der Knochennagel bzw. intramedulläre Nagel eine Öffnung auf, durch welche die Knochenschraube quer durch den Knochennagel geführt ist, um in einen Femurkopf einzugreifen. Die Knochenschraube ist dabei in der Öffnung so gelagert, dass die Knochenschraube gleiten kann. Um eine Bewegung der Knochenschraube einzuschränken, beispielsweise deren Rotation oder eine Bewegung der Knochenschraube in laterale Richtung, ist eine zusätzliche Stellschraube vorgesehen, welche mit der Knochenschraube interagiert. Die Stellschraube kann durch dieselbe Öffnung wie die Knochenschraube oder gegebenenfalls auch eine davon gesonderte Öffnung des Knochennagels geführt sein, ist aber im Knochennagel über ein Innengewinde in der Öffnung festgelegt.

Mehrteilige Sets zur Versorgung von, insbesondere proximalen, Femurfrakturen können beispielsweise gemäß der WO 2024/098081 A1 der Anmelderin so gestaltet sein, dass die Stellschraube durch eine Öffnung des Knochennagels verläuft, durch welche auch die Knochenschraube geführt ist. Die Stellschraube ist schräg zur Knochenschraube verlaufend angeordnet und interagiert mit dieser. Zu diesem Zweck können an der im Grundsatz zylindrisch ausgeformten Knochenschraube außenseitig mehrere Nuten eingeformt sein, welche konisch zulaufen. In diese konisch zulaufenden Nuten greift je nach Positionierung die schräg angestellte Stellschraube an, welche durch ein Gewinde festgelegt ist. Die Knochenschraube kann dann in eine laterale Richtung wandern, bis die konisch zulaufende Nut durch die festgelegte Stellschraube blockiert wird. Gleichzeitig wird eine Bewegungsmöglichkeit der Knochenschraube in Bezug auf eine Rotation und damit auch eine Verdrehung des Femurkopfes verhindert, da die Nut der Knochenschraube und damit auch die Knochenschraube nicht um die im Berührungsvolumen eingreifende Stellschraube gedreht werden kann. Solche Sets sind auch derart weiterentwickelt worden, dass während einer Operation mit zusätzlichem Instrumentarium gegebenenfalls eine Apposition oder Kompression der zu versorgenden, getrennten Knochenteile erfolgen kann.

Wenngleich Sets wie aus der WO 2024/098081 A1 bekannt, gegebenenfalls zusammen mit weiterem Instrumentarium für eine Apposition und/oder Kompression, bereits relativ einfach zu bedienen sind und viele Möglichkeiten bieten, hat sich doch gezeigt, dass es während einer Operation dazu kommen kann, dass die bereits positionierte Knochenschraube sich in einer bei der Einbringung der Stellschraube trotz vorheriger Ausrichtung an beispielsweise Markierungen in einer nicht vollständig korrekten Position befindet. Dies kann dazu führen, dass zunächst ein Bohrer, mit welchem ein Bohrloch für die Stellschraube zu bohren ist, nicht an der Knochenschraube vorbeigeführt werden kann. Selbst wenn ein Bohrloch noch einbringbar ist, kann eine falsche positionelle Ausrichtung der Knochenschraube dazu führen, dass die Stellschraube nicht in jenes Volumen eingreifen bzw. mit einem medialen Ende zu liegen kommen kann, welches durch eine Nut der Knochenschraube gebildet wird. In derartigen Fällen muss im aufwendigsten Fall die Knochenschraube wieder entnommen und der Vorgang von Neuem gestartet werden. Dies ist nicht nur bei dem gegebenen Knochenmaterial nachteilig, sondern auch für ein Operationsteam herausfordernd und erhöht zudem das Risiko möglicher Komplikationen wegen einer nicht optimalen Versorgung der Fraktur. Selbst bei einer einfacheren Behebung des Problems muss bei gesetzter Stellschraube zumindest der Stellschraubendreher samt Führungshülse wieder entnommen werden, um die Knochenschraube geeignet einzustellen, was allerdings nicht zwingend zum Erfolg führen muss.

Hier setzt die Erfindung an. Aufgabe der Erfindung ist es, ein Werkzeug der eingangs genannten Art anzugeben, welches erlaubt, auf einfache Weise die Knochenschraube vor der Einführung der Stellschraube ohne Entnahme der Knochenschraube nachzujustieren, sofern dies erforderlich ist.

Ein weiteres Ziel der Erfindung ist, ein Set der eingangs genannten Art derart weiterzubilden, dass ein Nachjustieren einer bereits befestigten Knochenschraube, insbesondere bei Befestigung in einem Femurkopf, auf einfache Weise erlaubt.

Ein weiteres Ziel der Erfindung ist es, ein Verfahren zur Versorgung von Knochenbrüchen, insbesondere proximalen Femurfrakturen, wobei eine Knochenschraube und eine mit der Knochenschraube zusammenwirkende Stellschraube durch eine Öffnung eines in einem Knochenteil angeordneten Knochennagels geführt werden, sodass die Stellschraube schräg zur Knochenschraube verläuft, wobei die Stellschraube eingerichtet ist, mit der Knochenschraube in Kontakt gebracht zu werden, um zumindest eine Bewegungsmöglichkeit der Knochenschraube festzulegen, derart weiterzubilden, dass die Knochenschraube nach deren Befestigung in einem Knochenteil, insbesondere einem Femurkopf, auf einfache Weise und möglichst sicher für ein Operationsteam in eine korrekte Position für die Einführung der Stellschraube bringbar ist.

Die Aufgabe der Erfindung wird bei einem Werkzeug der eingangs genannten Art gelöst, wenn der Verbindungsbereich eine Nut aufweist.

Bei einem erfindungsgemäßen Werkzeug ist von Vorteil, dass dieses auf einfache Weise eine korrekte Ausrichtung der Knochenschraube in Relation zu der erst später (leicht) schräg zur Knochenschraube verlaufenden Stellschraube ermöglicht. Grundsätzlich ist die Knochenschraube nach deren Positionierung, insbesondere in einem Femurkopf, bereits so ausgerichtet, dass die leicht schräg zu einer Längsachse der Knochenschraube einzubringende Stellschraube geeignet mit der Knochenschraube interagieren kann. Für ein Anordnen der Knochenschraube sowie der Stellschraube werden geeignete Führungshülsen in eine sogenannte Masterhülse eingebracht, in welcher bereits Gewebeschutzhülsen positioniert sind. Je nach Operationsgang werden in die Masterhülse samt Gewebeschutzhülsen geeignete Führungshülsen beispielsweise zunächst für Bohrdrähte und später dann für die Knochenschraube sowie einen Bohrer für das Bohren eines Lochs für die Stellschraube eingebracht. Ist die Knochenschraube über den Bohrdraht für die Knochenschraube geführt und eingebracht worden, kann es beispielsweise durch Umlagerung des Patienten oder andere Vorgänge dazu kommen, dass sich einzelne der Hülsen verschieben und/oder verdrehen, da für diese Hülsen eine gewisse Toleranz von wenigen Grad (üblicherweise weniger als 5°) gegeben ist. Dies kann allerdings bereits ausreichend sein, dass die Knochenschraube in Relation zu einer Hülse für den Bohrer für die Stellschraube nicht mehr korrekt ausgerichtet ist. Soweit der Bohrer betroffen ist, kann dies dazu führen, dass der Bohrer nicht an der Knochenschraube vorbeiführbar ist. Selbst wenn jedoch der Bohrer noch vorbeigeführt werden kann, kann es in der Folge dazu kommen, dass die Stellschraube ungewollt in einem lateralen Bereich bereits an die Knochenschraube anstößt und nicht, wie vorgesehen, in ein Berührungsvolumen eingreifen kann, das durch eine der mehreren außenseitig angeordneten, bevorzugt äquidistant am Umfang angeordneten, Nuten der Knochenschraube gebildet ist. Durch das Werkzeug ist es nun möglich, die Knochenschraube leicht zu bewegen, insbesondere zu rotieren, wobei diese im Knochenteil wie einem Femurkopf befestigt bleibt. Dies kann so lange erfolgen, bis der Bohrer oder die Stellschraube in die vom Werkzeug vorgegebene Nut, die distal der Knochenschraube liegt, in die distal frei zugängliche Nut des Werkzeuges einlaufen kann. Das Werkzeug ist dabei so eingerichtet, dass dieses die Knochenschraube derart fasst, dass mit der Positionierung des Werkzeuges an der Knochenschraube auch die Position der zumindest einen Nut der Knochenschraube (oder gegebenenfalls eine andere Art eines zusammenwirkenden Bereiches wie ein Anschlag) relativ zum Werkzeug festgelegt ist. Dadurch wird sichergestellt, dass mit dem Einlaufen des Bohrers oder der Stellschraube in die distal angeordnete Nut des Werkzeuges auch die korrekte Positionierung in Relation zur außenseitigen Nut der Knochenschraube gegeben ist. Durch diese konstruktiv einfache Maßnahme kann die Knochenschraube durch leichtes händisches Bewegen mit dem Werkzeug korrekt ausgerichtet werden, wenn sich trotz zuvor erfolgter fluchtender Ausrichtung der Markierungen an einer Gewebeschutzhülse und der Knochenschraube und/oder einem Schraubendreher für die Knochenschraube eine Blockade für den Bohrer und/oder die Stellschraube zeigt. Darüber hinaus kann das Werkzeug trotz der engen Platzverhältnisse bei der Einbringung von zwei zusammenwirkenden Schrauben (Knochenschraube und Stellschraube) während weiterer Operationsschritte, insbesondere eines Bohrens für die Stellschraube sowie des Einbringens der Stellschraube, an der Knochenschraube befestigt bleiben. Dies erlaubt es auch, am Werkzeug weitere Funktionalitäten vorzusehen, wie diese später noch erläutert werden.

Eine Befestigung des Werkzeuges an der Knochenschraube kann beispielsweise über einen medialen Ansatz erfolgen, der formschlüssig in ein laterales Ende der Knochenschraube eingreift. Dadurch kann insbesondere eine Nut des Verbindungsbereiches relativ zu einer Nut an der Knochenschraube festgelegt werden, da sich je nach Symmetrie des Formschlusses nur vorgegebene Positionen realisieren lassen. Zusätzlich kann innerhalb des Verbindungsbereiches ein Konnektivitätsmittel zur lösbaren lateralen Fixierung der Knochenschraube vorgesehen sein. Bei dem Konnektivitätsmittel kann es sich insbesondere um eine durch den Verbindungsbereich führbare hohle Stange handeln. Diese Stange kann an einem medialen Ende ein Gewinde, insbesondere ein Außengewinde, aufweisen, das mit einem lateralen Gewinde der Knochenschraube, insbesondere einem Innengewinde, zusammenwirkt. An einem lateralen Ende kann das Konnektivitätsmittel ein verbreitertes Ende aufweisen, mit dem das Konnektivitätsmittel wie eine Stange drehbar ist, um eine Verbindung zur Knochenschraube herzustellen.

Die Nut ist bevorzugt längserstreckt zwischen einem lateralen Ende und einem medialen Ende verlaufend ausgebildet. Die Nut kann einen Querschnitt aufweisen, welcher an einen Außendurchmesser des Bohrers für die Stellschraube sowie die Stellschraube und/oder eine Führungshülse angepasst ist. In der Regel ist die Nut mit einem kreissektorförmigen Querschnitt ausgebildet. Dieser Querschnitt kann in mediale Richtung hin betrachtet verbreitert ausgeführt sein, weil die Stellschraube nicht parallel zur Knochenschraube, sondern unter leichter Schrägstellung (Winkel von bis zu 20°, bevorzugt bis zu 15°, insbesondere 10° oder weniger, beispielsweise weniger als 3°) zur Knochenschraube verläuft. Durch eine entsprechende Ausbildung der Nut des Werkzeuges wird dieser Schrägstellung ausreichend Rechnung getragen. Somit kann die Nut relativ zu einer Befestigungsachse schräg verlaufen. Die Befestigungsachse der Knochenschraube koinzidiert mit einer Längsachse derselben. Relativ zur Befestigungsachse bzw. Längsachse ergibt sich der erwähnte Winkel, mit dem die Stellschraube relativ zur Knochenschraube schräg verläuft.

Die Nut im bzw. am Verbindungsbereich kann mit einer Länge ausgebildet sein, die zumindest ein Drittel, bevorzugt zumindest die Hälfte einer Länge des Verbindungsbereiches beträgt. Die Nut kann quer zu deren Längsachse eine maximale Breite aufweisen, die zumindest 60 %, bevorzugt zumindest 70 %, insbesondere 80 % oder mehr, eines Durchmessers des Verbindungsbereiches quer zu einer Längsachse desselben beträgt.

Der Verbindungsbereich ist bevorzugt schaftförmig ausgebildet. Der Verbindungsbereich ist von der Länge eingerichtet, eine Distanz zu einer bereits gesetzten Knochenschraube zu überbrücken. Demensprechend ist der Verbindungsbereich bevorzugt als länglicher, sich vom Grundkörper des Werkezuges in mediale Richtung hin erstreckender Werkzeugteil ausgebildet. Insbesondere kann der Verbindungsbereich etwa schaftförmig ausgebildet sein. Gegebenenfalls kann der Verbindungsbereich in dessen schaftförmigen Anteil hohl ausgebildet sein, insbesondere um durch ein hohles Inneres des Verbindungsbereiches ein Befestigungsinstrument einzuführen, mit welchem die Knochenschraube lösbar befestigt werden kann.

Besonders bevorzugt weist der Grundkörper am zweiten Ende eine Aufnahme zum Aufsetzen und Befestigen des Werkzeuges an einem insbesondere lateralen Ende einer Knochenschraube auf. Die laterale Befestigung ist aus Gründen einer Hantierbarkeit bevorzugt durch einen den Verbindungsbereich darstellenden länglichen Schaft des Werkzeuges möglich. Der Schaft kann mit dem Grundkörper dauerhaft verbunden sein, aber auch als separater, lösbar am Grundkörper befestigbarer Teil des Werkzeuges ausgebildet sein. Wie erläutert kann das Werkzeug dabei lösbar mit der Knochenschraube an einem lateralen Ende der Knochenschraube verbunden werden. Bei der Aufnahme kann es sich vorzugsweise um einen an einem lateralen Ende der Knochenschraube aufsetzbaren Ansatz handeln. Der medial angeordnete Ansatz greift mit Vorteil formschlüssig in ein laterales Ende der Knochenschraube ein, sodass der medial angeordnete Ansatz mit dem lateralen Ende der Knochenschraube formschlüssig zusammenwirkt. Zur Fixierung einer Verbindung des Werkzeuges mit der Knochenschraube in Translationsrichtung zwischen medial und lateral kann ein durch den hohlen Schaft geführtes Befestigungsinstrument dienen, das sich an einem lateralen Ende des Werkzeuges einführen und dort betätigen lässt, um die Knochenschraube neben dem formschlüssigen Ansatz auch in Bezug auf eine Translation zwischen lateral und medial zu fixieren. Hierbei kann es sich insbesondere um das vorstehend erwähnte Konnektivitätsmittel handeln. Grundsätzlich ist es aber auch möglich, dass eine Verbindung der Knochenschraube mit dem Grundkörper über eine andere, gesonderte Werkzeugkomponente hergestellt wird.

Der Grundkörper, der üblicherweise aus einem Stahl gebildet ist, insbesondere aus einem nicht rostenden Stahl, kann eine Justierschraube umfassen, wobei die Justierschraube parallel zur Befestigungsachse verläuft. Die Justierschraube kann beispielsweise in Form einer Madenschraube ausgebildet sein. Die Justierschraube ist mit Vorteil durch den Grundkörper geführt. Dabei kann insbesondere vorgesehen sein, dass der Grundkörper ein Innengewinde aufweist, in welches ein Außengewinde der Justierschraube eingreift, wobei die Justierschraube länger ausgebildet ist als eine Distanz zwischen einem lateralen Rand und einem medialen Rand im Bereich der Justierschraube und entlang der Befestigungsachse. Mit anderen Worten ist die Justierschraube länger ausgebildet als die erwähnte Distanz zwischen dem lateralen Rand und dem medialen Rand. Auf Grundlage des Innengewindes im Grundkörper sowie des damit zusammenwirkenden Außengewindes der Justierschraube kann die Justierschraube durch den Grundkörper geschraubt werden. Die Justierschraube ist somit relativ zum Grundkörper bewegbar, oder umgekehrt betrachtet der Grundkörper zur Justierschraube, wenn die Justierschraube an einem Anschlag ansteht und betätigt wird. Genau dies kann genutzt werden, um das Werkzeug samt daran unmittelbar oder mittelbar befestigter Knochenschraube in laterale Richtung zu bewegen, wenn ein mediales Ende der Justierschraube an einem Teil des Sets für die Operation ansteht, beispielsweise einer Gewebeschutzhülse. Wird die Justierschraube dann betätigt, wird das Werkzeug samt Knochenschraube in laterale Richtung geschoben. Dadurch ist eine Apposition der getrennten Knochenteile möglich. Eine laterale Maximalbewegung wird dabei durch einen Eingriff der Stellschraube limitiert. Besonders bevorzugt ist es dabei, dass die Justierschraube benachbart zum dem ersten Ende, insbesondere benachbart zum Verbindungsbereich, angeordnet ist. Damit sind die entscheidenden Achsen (Befestigungsachse entlang des Verbindungsbereiches bzw. Längsachse der Knochenschraube und Translationsachse der Justierschraube) nahe beieinander, was für eine sichere Translation des Werkzeuges samt Knochenschraube in laterale Richtung günstig ist. Gleichwohl könnte die Justierschraube auch an anderer Stelle des Werkzeuges positioniert sein, so lange ein erforderlicher Anschlag an einem Teil des Sets gefunden wird, um die Relativbewegung zwischen Justierschraube und den übrigen Teilen des Werkzeuges unter gleichzeitiger Verschiebung Letzterer zu ermöglichen.

In einer Weiterbildung kann vorgesehen sein, dass das Werkzeug eine Arretierungseinrichtung zur lösbaren Befestigung des Werkzeuges an einem weiteren Teil des Sets umfasst. Hierbei kann es sich um ein Betätigungsmittel für die Stellschraube handeln, beispielsweise einen Schraubendreher. Das Betätigungsmittel weist bevorzugt einen Anschlag auf, an dem die Arretierungseinrichtung von medial nach lateral betrachtet nachgeordnet zur Anlage kommen kann. Wird die Arretierungseinrichtung in Richtung von medial nach lateral nach dem Anschlag angeordnet und ist das Werkzeug gleichzeitig mit der Knochenschraube verbunden, führt dies zunächst zu einer Verriegelung zwischen der Betätigungseinrichtung für die Stellschraube und die in der Öffnung des Knochennagels gleitbar gelagerte Knochenschraube. Wird nun die Stellschraube, deren Außengewinde in ein Innengewinde der Öffnung des Knochennagels eingreift, mit der Betätigungseinrichtung wie einem Schraubendreher gelöst, also durch Linksdrehung nach lateral bewegt, nimmt der Anschlag die Arretierungseinrichtung des Werkzeuges und damit das Werkzeug und in der Folge auch die Knochenschraube in die laterale Richtung mit. Selbst wenn die Stellschraube an sich wie bei der zuvor erläuterten Apposition eine weitere laterale Bewegung der Knochenschraube bereits verhindern würde, ist dies bei diesem Vorgang nicht der Fall, da in diesem Fall auch die Stellschraube in laterale Richtung bewegt wird, somit der gesamte Verbund aus Knochenschraube und Stellschraube. Damit ist es möglich, die voneinander getrennten Knochenteile aneinander anzupressen und dadurch zu komprimieren. Somit ist die Funktionalität des Werkzeuges nochmals erweitert, sodass es sich letztlich um ein multifunktionales Werkzeug mit folgenden Möglichkeiten handelt:
- korrekte Ausrichtung der Knochenschraube für das Einbringen der Stellschraube;
- Durchführung einer Apposition;
- Durchführung einer Kompression.

Gleichzeitig ist das Werkzeug nicht nur einfach ausgebildet, sondern auch einfach handhabbar und bietet kaum Möglichkeiten für Fehler. Das Werkzeug braucht während der einzelnen Schritte auch nicht abgenommen werden, sondern kann für die Durchführung der vorstehend angeführten Funktionalitäten befestigt bleiben, was zusätzlich die Handhabbarkeit verbessert und zur Fehlerreduktion beiträgt.

Die Arretierungseinrichtung kann insbesondere im Greifbereich angeordnet sein. Bevorzugt ist die Arretierungseinrichtung zwischen erstem Ende und zweitem Ende längsverschiebbar gelagert. Dabei kann das Werkzeug einen Freigabe- und Fixiermechanismus für die Arretierungseinrichtung umfassen. In einer derartigen konstruktiven Gestaltung ist es auf einfache Weise möglich, die Arretierungseinrichtung zunächst freizugeben und längs zu verschieben, also von einer proximalen in eine distale Position. In dieser distalen Position kann die Arretierungseinrichtung am erwähnten Anschlag der Betätigungseinrichtung für die Stellschraube zur Anlage kommen. Gegebenenfalls kann in dieser Position wieder arretiert werden, solange dies erforderlich ist. Später kann die Arretierungseinrichtung gelöst und wieder in die proximale Ausgangsposition verschoben werden. Somit ist eine Längsverschiebbarkeit der Arretierungseinrichtung bevorzugt quer zur Befestigungsachse und damit einer Längsachse der Knochenschraube gegeben und die Arretierungseinrichtung quer zur Befestigungsachse verschiebbar gelagert.

Der Grundkörper ist mit Vorteil im Wesentlichen eben ausgebildet. Im Wesentlichen eben bedeutet dabei, dass der Grundkörper manuell ergriffen werden kann, die Ausbildung entlang der Erstreckung vom ersten Ende zum zweiten Ende aber im Wesentlichen in Draufsicht mit gleicher Dicke gegeben ist. Dadurch ist der Grundkörper gut hantierbar, bringt die gewünschten und zuvor erläuterten Funktionalitäten mit und stellt dennoch kein Hindernis bei Operationsschritten dar, welche nach der Befestigung des Werkzeuges erfolgen. Am Grundkörper kann im Bereich des zweiten, insbesondere distalen Endes ein Schaft angeordnet sein, der mit der Knochenschraube interagieren kann, insbesondere mit dem lateralen Ende der Knochenschraube. Hierfür kann am Schaft an einem freien, medial eingreifenden Ende ein Außengewinde vorgesehen sein, das mit einem endseitigen, lateralen Innengewinde der Knochenschraube zusammenwirkt.

Das Werkzeug wird bevorzugt auch zum Eindrehen der Knochenschraube an sich genutzt, übernimmt also auch die Funktion eines Schraubendrehers.

Das weitere Ziel wird durch ein Set der eingangs genannten Art erreicht, wenn das Set ein Werkzeug zur insbesondere rotierenden Bewegung der Knochenschraube im befestigten Zustand umfasst, insbesondere ein erfindungsgemäßes Werkzeug wie zuvor erläutert, wobei das Werkzeug eine Nut für die Stellschraube aufweist.

Mit einem derartigen Set kann ein Operationsinstrumentarium zur Einbringung eines Implantats in Form eines Knochennagels sowie einer damit zusammenwirkenden Knochenschraube und einer Stellschraube bereitgestellt werden, welches sich als vorteilhaft für eine möglichst fehlerfreie Operation mit rascher Positionierung der Knochenschraube in einer korrekten Position zur Aufnahme der Stellschraube eignet. Ist das vorgesehene Werkzeug erfindungsgemäß ausgebildet, kann mit dem Set gegebenenfalls auch eine Apposition (Annäherung der Flächen gebrochener Knochenteile aneinander) sowie gegebenenfalls auch eine Kompression (Anpressen voneinander getrennter Knochenteile) erfolgen. Dabei kann das Werkzeug vom Schritt einer allfälligen Repositionierung der Knochenschraube vor Einbringung der Stellschraube über eine Apposition bis hin zu einer Kompression an der Knochenschraube befestigt bleiben. Dies ist deswegen bemerkenswert, weil an sich die Platzverhältnisse im Bereich der lateralen Aufnahme der Masterhülse, über welche sowohl die Knochenschraube als auch die leicht schräg angestellte Justierschraube eingebracht werden, sehr beschränkt sind.

Die Stellschraube kann wie erwähnt in einem Winkel schräg zur Knochenschraube verlaufend im Knochennagel fixierbar sein. Für eine Schrägstellung ist es ausreichend, wenn der Winkel relativ klein ist. Winkel von < 10°, vorzugsweise < 5°, insbesondere < 3° sind in der Regel ausreichend.

Die Stellschraube ist eingerichtet, in ein durch die Knochenschraube definiertes Berührungsvolumen einzugreifen. Ein Berührungsvolumen kann durch eine oder mehrere Nuten an der Knochenschraube vorgegeben sein. Die Nut oder die Nuten sind dabei außenseitig an der Knochenschraube angeordnet. Die Knochenschraube ist im Wesentlichen zylindrisch ausgebildet. Die zylindrische Ausbildung kann mit einem kreisförmigen Querschnitt gegeben sein. Die zylindrische Ausbildung der Knochenschraube erfolgt mit einer Längserstreckung entlang einer Längsachse der Knochenschraube. An einem medialen Ende weist die Knochenschraube ein Außengewinde auf, das in einem Knochenteil eingreift. An einem lateralen Ende weist die Knochenschraube in der Regel eine vertieft angeordnete geometrische Struktur zum Einsetzen eines Schraubendrehers oder eines anderen Betätigungsmittels ein, mit welchem die Knochenschraube in Rotation um deren Längsachse versetzt werden kann, damit die Knochenschraube in einem Knochenteil nach Setzen eines Bohrlochs einschraubbar ist. Am lateralen Ende kann die Knochenschraube des Weiteren eine von der Zylinderform abweichende asymmetrische Form aufweisen, sodass an dieser Stelle das Werkzeug mit der Aufnahme lediglich in einer einzigen Position aufgesetzt werden kann. Dadurch sind falsche Montagen des Werkzeuges an der Knochenschraube vermieden und ist sogleich eine relative Ausrichtung der Nut oder Nuten der Knochenschraube relativ zum Werkzeug gegeben. Die Nut oder die Nuten definieren ein oder mehrere Berührungsvolumina, in welche die Stellschraube eingreifen kann. Dadurch, dass die Stellschraube insbesondere mit einem medialen Ende in ein Volumen eingreift, welches durch eine Nut gebildet wird, ist zunächst eine signifikante Rotation der Knochenschraube samt Femurkopf vermieden, da die Nut dann an einem medialen Ende der Stellschraube anschlägt. Somit wird eine Bewegung der Knochenschraube im Sinne einer Rotation derselben um eine von der Längsachse der Knochenschraube verschiedene Rotationsachse unterbunden. Soweit eine laterale Bewegung der Knochenschraube betroffen ist, kann diese durch die Stellschraube ebenfalls entweder vollständig unterbunden oder in vorbestimmten Maßen freigegeben sein. Da die Nut oder die Nuten an der Knochenschraube üblicherweise konisch zulaufend ausgebildet sind, und zwar von einer lateralen Seite zu einer medialen Seite hin, kann sich die Knochenschraube bei geeigneter Justierung der Stellschraube so lange nach lateral bewegen, bis die konisch immer schmäler werdende Nut der Knochenschraube schließlich durch die Stellschraube blockiert wird, was eine weitere laterale Bewegung der Knochenschraube unterbindet. Möglich ist es aber auch, dass eine laterale Verschiebung der Knochenschraube während des Heilungsprozesses vollständig unterbunden wird, indem die Stellschraube sofort auf Anschlag gebracht wird. Neben der Ausbildung mit einer konischen Nut der Knochenschraube kommen allerdings auch andere Varianten zur Anwendung, welche bestimmte laterale Bewegungen der Knochenschraube ermöglichen, beispielsweise eine stufenförmige Ausbildung einer Nut. Im Berührungsvolumen muss die Stellschraube nicht zwingend die Knochenschraube anfänglich berühren, sondern es ist ausreichend, dass die Stellschraube, insbesondere ein mediales Ende derselben, in dieses Volumen einragt.

Das erfindungsgemäße Set kann zudem Bohrdrähte, eine Masterhülse und eine oder mehrere Gewebeschutzhülsen, diverse Führungshülsen, ein Zielgerät für den Knochennagel und Gerätschaften wie Schraubendreher umfassen.

Das verfahrensmäßige Ziel wird erreicht, wenn bei einem Verfahren der eingangs genannten Art die Knochenschraube zur Positionierung der Stellschraube mit einem Werkzeug, insbesondere einem erfindungsgemäßen Werkzeug, bewegt wird.

Bei einem erfindungsgemäßen Verfahren ist von Vorteil, dass sich auf einfache Weise die Knochenschraube bei einem allfälligen Verrücken einer Gewebeschutzhülse, und damit der für die korrekte Ausrichtung der Knochenschraube an sich erforderliche Markierung, die Knochenschraube einfach, rasch und präzise in eine korrekte Position bringen lässt, sodass sowohl der Bohrer für die Stellschraube als auch die Stellschraube selbst korrekt eingeführt werden können. Dabei wird im Rahmen des Verfahrens mit Vorteil ein erfindungsgemäßes Set eingesetzt, welches wiederum mit Vorteil ein erfindungsgemäßes Werkzeug umfasst.

Im Rahmen des Verfahrens kann insbesondere vorgesehen sein, dass die Knochenschraube mit dem Werkzeug im Wesentlichen durch Rotation bewegt wird. Beim Einschrauben kann auch eine Translation quer zu einer Längsachse der Knochenschraube erfolgen.

Es kann in einem Verfahrensschritt vorgesehen sein, dass mit dem Werkzeug die Knochenschraube erfasst und mit einer Justierschraube des Werkzeuges in laterale Richtung bewegt wird. Dadurch ist bei gleichzeitig feststehender Stellschraube eine Apposition möglich.

Des Weiteren kann im Rahmen des Verfahrens vorgesehen sein, dass mit dem Werkzeug die Knochenschraube erfasst und das Werkzeug an einem mit der Stellschraube in Verbindung stehenden oder durch dieses selbst gebildeten Anschlag angestellt wird, wonach die Knochenschraube durch das Werkzeug bewegt wird, indem durch einen medialen Anschlag eines Stellmittels des Werkzeuges wie eine Justierschraube ein Teil des Werkzeuges samt der damit verbundenen Knochenschraube in laterale Richtung verschoben wird. Dadurch ist eine Kompression, also ein Anpressen der voneinander getrennten Knochenteile möglich.

Weitere Merkmale, Vorteile und Wirkungen der Erfindung ergeben sich aus den nachfolgend dargestellten Ausführungsbeispielen sowie den Erläuterungen zur Durchführung einer Operation mit einem erfindungsgemäßen Werkzeug sowie einem Set, in welchem das Werkzeug Anwendung findet. In den Zeichnungen, auf welche dabei Bezug genommen wird, zeigen:
Fig. 1 eine Seitenansicht eines erfindungsgemäßen Werkzeuges, befestigt auf einer Knochenschraube;
Fig. 2 eine stirnseitige Ansicht des Werkzeuges gemäß Fig. 1 samt Knochenschraube;
Fig. 3 eine Draufsicht auf das Werkzeug samt Knochenschraube gemäß Fig. 1;
Fig. 4 eine rücksichtige Ansicht des Werkzeuges samt Knochenschraube gemäß Fig. 1;
Fig. 5 eine Unteransicht auf das Werkzeug samt Knochenschraube gemäß Fig. 1;
Fig. 6 das Werkzeug gemäß Fig. 1 bis Fig. 5 in einer perspektivischen Ansicht, jedoch ohne Knochenschraube;
Fig. 7 bis Fig. 11 die Situation gemäß Fig. 1 bis Fig. 5 während eines Operationsschrittes;
Fig. 12 das Werkzeug gemäß Fig. 7 bis Fig. 11 in einer perspektivischen Darstellung;
Fig. 13 einen Ausschnitt eines Sets zur Versorgung einer proximalen Femurfraktur mit befestigtem Werkzeug;
Fig. 14 das Set gemäß Fig. 13 während der Durchführung einer Apposition von getrennten Knochenteilen;
Fig. 15 das Set aus Fig. 13 und Fig. 14 während der Durchführung einer Kompression von getrennten Knochenteilen;
Fig. 16 eine perspektivische Ansicht eines Sets während eines Operationsschrittes.

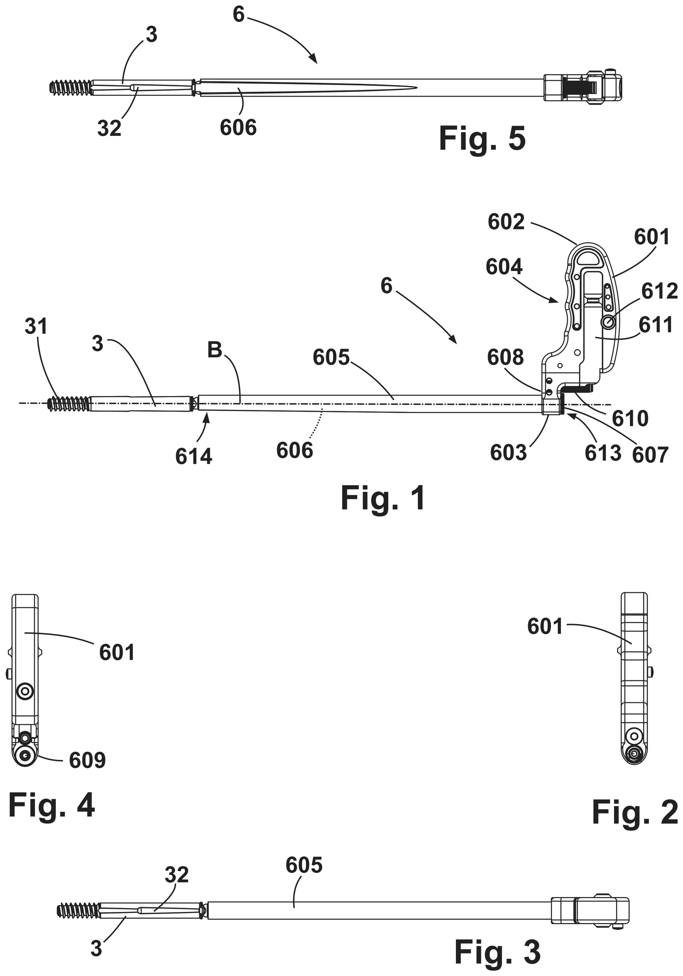

In Fig. 1 bis Fig. 4 ist ein erfindungsgemäßes Werkzeug 6 dargestellt. Wie in Fig. 1 ersichtlich ist, ist das Werkzeug 6 auf einer Knochenschraube 3 angeordnet, deren Funktion nachstehend noch erläutert wird. Die Knochenschraube 3 ist im Wesentlichen zylindrisch und länglich ausgebildet und weist eine Längsachse auf, welche mit einer Befestigungsachse B koinzidiert. Die Knochenschraube 3 ist dazu eingerichtet, in einen Knochenteil eingebracht bzw. in diesem fixiert zu werden, insbesondere einem Femurkopf, und weist dafür medial endseitig ein Außengewinde 31 auf.

Wie aus Fig. 1 bis Fig. 5 ersichtlich ist, weist das Werkzeug 6 einen Grundkörper 601 auf, der aus einem Stahl, insbesondere einem nicht rostenden Stahl gefertigt sein kann. Der Grundkörper 601 weist ein erstes Ende 602 und ein zweites Ende 603 auf, wobei aufgrund der Darstellung in Fig. 1 von oben nach unten etwa länglichen Ausbildung des Grundkörpers 601 das zweite Ende 603 dem ersten Ende 602 gegenüberliegt. Das erste Ende 602 kann als erstes, proximales Ende 602 und das gegenüberliegende zweite Ende 603 als zweites, distales Ende 603 angesehen werden. Der Grundkörper 601 ist, wie insbesondere aus Fig. 2, aber auch der Zusammenschau aus Fig. 1 bis Fig. 5 ersichtlich ist, im Wesentlichen eben ausgebildet. Eine gewisse Dicke ergibt sich aus dem Erfordernis, dass der Grundkörper 601 für Operationspersonal gut greifbar sein soll. Hierfür weist der Grundkörper 601 einen Greifbereich 604 auf, welcher dem ersten

Ende 602 benachbart angeordnet ist bzw. an dieses anschließt und sich in distale Richtung zum zweiten Ende 603 hin erstreckt. **Im** Greifbereich 604 ist das Werkzeug 6 nicht nur mit im Wesentlichen konstanter Dicke (wie auch in den übrigen Bereichen), sondern auch im Wesentlichen mit konstanter Breite ausgebildet, wie dies in Fig. 1 in der Seitenansicht ersichtlich ist. An einer medialen Stirnseite des Greifbereiches 604 ist eine Wellenform vorgesehen, welche ein manuelles Greifen im Greifbereich 604 mit einer menschlichen Hand und damit einen sicheren Halt des Werkzeuges 6 für Operationspersonal erleichtert. An dem Greifbereich 604 schließt ein Verbindungsbereich 605 an, der am zweiten Ende 603 eine Aufnahme 609 aufweist, die in Fig. 2 und Fig. 4 ersichtlich ist. Diese Aufnahme 609 ist derart ausgebildet, dass diesen den Verbindungsbereich 605 aufnimmt. Die entsprechende Verbindung zwischen Grundkörper 601 und Verbindungsbereich 605 kann lösbar oder dauerhaft sein. Über ein mediales Ende des Verbindungsbereiches 605 kann das Werkzeug 6 formschlüssig auf ein laterales Ende der Knochenschraube 3 aufgeschoben werden. Da die Knochenschraube 3 an deren lateralen Ende mit einer Vertiefung mit einer Symmetrie entsprechend einer mehrzähligen Drehachse ausgebildet ist (zum Beispiel ein Vieleck wie ein Dreieck oder Fünfeck oder Sechseck) und das mediale Ende des Verbindungsbereiches 605 hierzu korrespondierend geformt ist, besteht vorzugsweise lediglich die Möglichkeit, den Verbindungsbereich 605 mit dem lateralen Ende der Knochenschraube 3 so zu verbinden, dass es zu keinen falschen Anordnungen des Werkzeuges 6 an der Knochenschraube 3 kommen kann. Mit Vorteil erstreckt sich die Aufnahme 609 als durchgehende Öffnung zwischen einem lateralen Rand 607 und einem medialen Rand 608 des Werkzeuges 6, welchen den Verbindungsbereich 605 aufnimmt, der im Wesentlichen schaftförmig oder als Schaft ausgebildet ist. Wie in der Seitenansicht in Fig. 1 ersichtlich ist, ist das Werkzeug 6 dabei im Verbindungsbereich 605 im Vergleich mit dem Greifbereich 604 deutlich länger ausgebildet. Insbesondere ist der Verbindungsbereich 605 derart lang ausgebildet, dass die Knochenschraube 3 in gewünschter Weise in einem Knochen befestigt und mit dem Werkzeug 6 manipuliert werden kann. Des Weiteren ist der Greifbereich 604 relativ zum Verbindungsbereich 605 lateral rückspringend ausgebildet. Dadurch ist es möglich, das Werkzeug 6 mit gutem Sichtkontakt an der Knochenschraube 3 befestigen zu können, selbst wenn das Werkzeug 6 von einer kräftigen Hand erfasst ist. Anders ausgedrückt kann auch gesagt werden, dass der Verbindungsbereich 605 vorspringend zum Greifbereich 604 ausgebildet ist. Mit dem Greifbereich 604 und dessen relativ breiter Ausbildung und dem deutlich länger ausgebildeten Verbindungsbereich 605 wird auch eine ausreichend gute Kraftübertragung ermöglicht, sodass eine Rotation des Werkzeuges 6 und damit der damit in Verbindung stehenden Knochenschraube 3 mit einem geringen manuellen Kraftaufwand möglich ist.

Eine weitere Komponente des Werkzeuges 6 ist eine Justierschraube 610, die insbesondere als Madenschraube ausgebildet sein kann. Die Justierschraube 610 ist längserstreckt ausgebildet. Die Justierschraube 610 weist ein Außengewinde auf, welches mit einem Innengewinde zusammenwirkt, welches parallel zur Befestigungsachse B und damit auch zu einer Längsachse der Knochenschraube 3 sowie der Befestigungsachse B verläuft.

Des Weiteren weist das Werkzeug 6 im Bereich des Verbindungsbereiches 605 ein laterales Ende 613 und ein mediales Ende 614 und benachbart zum medialen Ende 614 von bevorzugt von diesem ausgehend eine Nut 606 auf, die insbesondere in Fig. 5 ersichtlich ist. Die distal angeordnete Nut 606 dient nach einer ersten Überlegung dazu, eine später einzuführende Stellschraube 4 bzw. bereits zuvor einen Bohrer zum Einbringen eines Loches für die mit der Knochenschraube 3 zusammenwirkende Stellschraube 4 freizustellen. In einer zweiten Überlegung, die auf der ersten Überlegung basiert, kann bei der erläuterten formschlüssigen Verbindung des Werkzeuges 6 mit dem lateralen Ende der Knochenschraube 3 auch ein korrektes Einlaufen der Stellschraube 4 sichergestellt werden. Durch eine einzig mögliche formschlüssige Verbindung des Werkzeuges 6 mit der Knochenschraube 3 an deren lateralen Ende ergibt sich nämlich, dass mit dem Werkzeug 6 auch die Lage der Nut 606 der Knochenschraube 3 festlegbar ist. Wenn die Abstimmung so getroffen ist, dass die Nut 606 des Werkzeuges 6 auch ein Einlaufen der Stellschraube 4 und zuvor des Bohrers für dieselbe ermöglicht, ist automatisch eine korrekte Positionierung der Knochenschraube 3 und einer von gegebenenfalls mehreren außenseitigen Nuten 32 gegeben, in welcher Nut 32 der Knochenschraube 3 die Stellschraube 4 in einem Berührungsvolumen insbesondere mit einem medialen Ende der Stellschraube 4 zu liegen kommt und dort gegebenenfalls durch Berührung mit einer Außenseite der Knochenschraube 3 eine Blockade einer Rotation der Knochenschraube 3 um eine von der Längsachse der Knochenschraube 3 verschiedene Längsachse und/oder eine laterale Bewegung der Knochenschraube 3 auf ein bestimmtes Maximalmaß einschränkt oder ganz verhindert.

Das Werkzeug 6 stellt somit in einer ersten Funktion ein Werkzeug 6 zur Verfügung, welches eine korrekte Ausrichtung der Knochenschraube 3 vor Einbringung der Stellschraube 4 ermöglicht. Dies kann insbesondere deswegen wichtig sein bzw. werden, weil aufgrund einer Umlagerung eines Patienten oder anderer Vorgänge die Knochenschraube 3 nicht jedenfalls korrekt positioniert sein muss. Üblicherweise ist an einer Gewebeschutzhülse 9 eine Markierung angebracht, ebenso wie an der Knochenschraube 3 und/oder einem Schraubendreher zum Einbringen der Knochenschraube 3. Die Markierung an der Knochenschraube 3 und gegebenenfalls deren Verlängerung am Werkzeug 6 werden in fluchtender Übereinstimmung mit der Markierung an die Gewebeschutzhülse 9 gebracht. Da dies durch Rotieren der Knochenschraube 3 erfolgt, sollte bei fluchtender Übereinstimmung der Markierungen die Knochenschraube 3 ausreichend genau zur Aufnahme bzw. zum Eingriff der Stellschraube 4 ausgerichtet sein. Es kann allerdings vorkommen, dass während einer Operation die Gewebeschutzhülse 9, in welcher die Knochenschraube 3 verläuft, um wenige Grad verdreht wird, da für die Anordnung der Gewebeschutzhülse 9 in einer Masterhülse 8 eine gewisse Toleranz gegeben ist. Wenngleich diese mit beispielsweise maximal 5° relativ gering ist, kann dies dennoch signifikante Auswirkungen haben, da eine schräge Anstellung der Stellschraube 4 einen noch geringeren Winkel von beispielsweise weniger als 1,5° aufweist, was zu einer unerwünschten Blockade bzw. einem Anschlagen der Stellschraube 4 außerhalb oder nicht im gewünschten Bereich des Berührungsvolumens führen kann. Dies führt während einer Operation zu Komplikationen. Bislang bestand eine Behebung derartiger Situationen zumeist darin, dass das System rückgebaut wurde, also die Knochenschraube 3 im schlechtesten Fall nochmals entnommen und neu positioniert wurde. Nunmehr kann mit dem erfindungsgemäßen Werkzeug 6 auf einfache Weise die Knochenschraube 3 geringfügig so lange rotiert werden, bis die Stellschraube 4 oder gegebenenfalls bereits zuvor der Bohrer für dieselbe Nut 606 des Werkzeuges 6 frei passieren kann und damit gleichzeitig die korrekte Ausrichtung einer außenseitigen Nut 32 der Knochenschraube 3 sichergestellt ist. Somit ist eine sofortige Korrektur möglich, wenn sich beim Einbringen der Stellschraube 4 unerwartet Komplikationen zeigen.

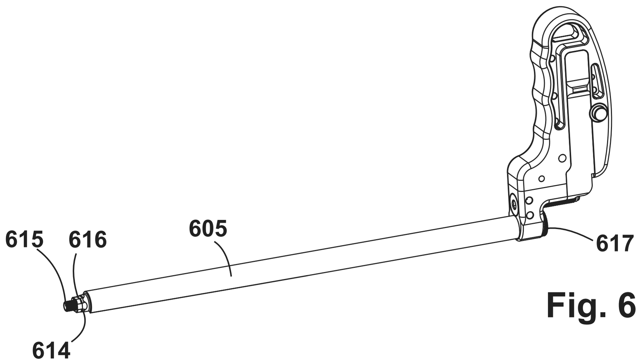

Die Knochenschraube 3 kann am Werkzeug 6 lösbar befestigt fixiert sein. Hierfür kann gemäß Fig. 6 innerhalb des hohlen Schaftes bzw. Verbindungsbereiches 605 ein Konnektivitätsmittel wie eine hohle Stange geführt sein. Das Konnektivitätsmittel kann einem medialen Ende ein Außengewinde 615 aufweisen, das in ein lateral an der Knochenschraube 3 angeordnetes Innengewinde der Knochenschraube 3 eingreift. Über die am medialen Ende 614 des Verbindungsbereiches 605 angeordnete Struktur 616 kann ein Formschluss mit des Verbindungsbereiches 605 mit der Knochenschraube 3 erreicht werden, sodass die Nut 606 mit einer der Nuten 32 der Knochenschraube 3 ausgerichtet ist. Über das durch den Verbindungsbereich 605 hindurchgeführte Konnektivitätsmittel erfolgt dann die lösbare Verbindung mit der Knochenschraube 3. Hierfür kann am Konnektivitätsmittel lateral endseitig ein Betätigungsrad 617 vorgesehen sein.

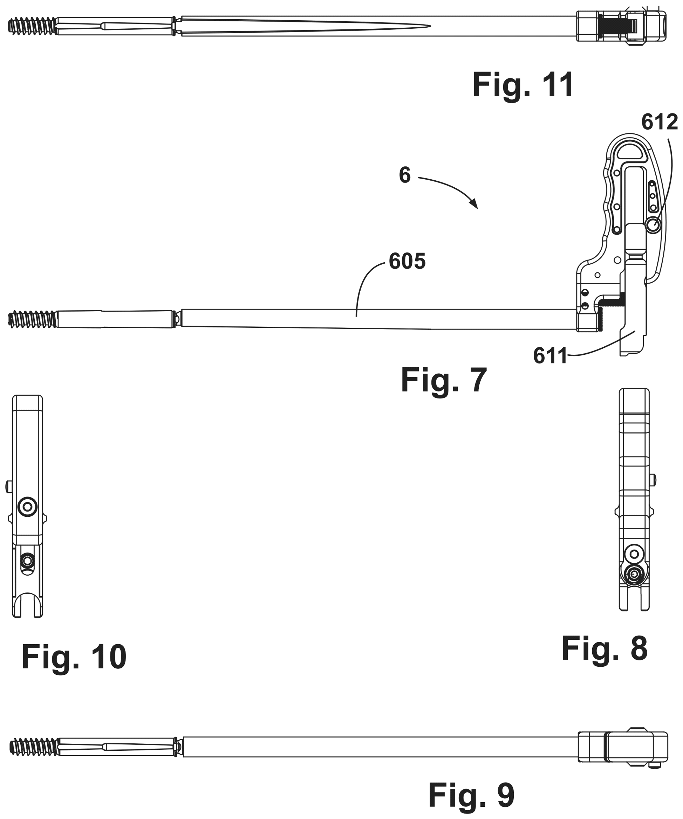

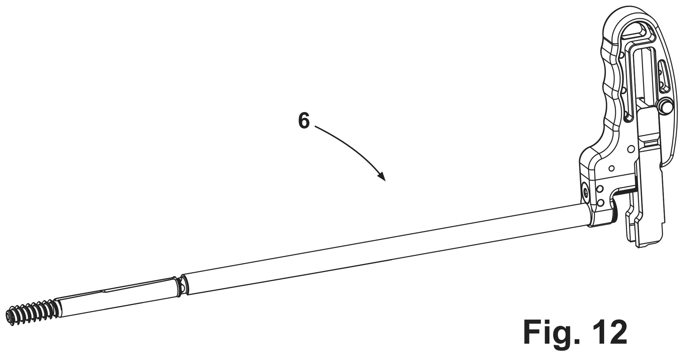

Mit der Justierschraube 610 sowie einer in Fig. 1 ersichtlichen Arretierungseinrichtung 611 lassen sich weitere funktionale Möglichkeiten schaffen. Wie in Fig. 1 ersichtlich ist, ist die Arretierungseinrichtung 611 als länglicher Schieber ausgebildet. Dieser längliche Schieber arbeitet mit einem Freigabe- und Fixiermechanismus 612 zusammen. Der Schieber kann wie zum Grundkörper 601 aus einem Metall oder einer Legierung bestehen, insbesondere um eine maximale Steifigkeit zu generieren. Der Freigabe- und Fixiermechanismus 612 kann zum Beispiel einen üblichen Mechanismus darstellen, welcher die Betätigung eines gleitbar gelagerten Elementes ermöglicht, wenn eine vorbestimmte Federkraft überwunden wird. Auch andere, einfache Verriegelungsmechanismen sind selbstverständlich möglich, so lange sichergestellt ist, dass der Schieber gemäß Fig. 1 bis Fig. 6 zunächst in einer Ausgangsposition gehalten wird, bei Betätigung des Freigabe- und Fixiermechanismus 612 aber von der Ausgangsposition in eine Betriebsposition geschoben werden kann, die in Fig. 7 bis Fig. 12 ersichtlich ist, oder automatisch in diese Betriebsposition springt, beispielsweise bei einer gefederten Lagerung, die freigegeben wird. Der Freigabe- und Fixiermechanismus 612 kann so ausgebildet sein, dass in dieser Betriebsposition der längliche Schieber ebenfalls fixiert ist oder fixiert werden kann, was aber nicht zwingend ist.

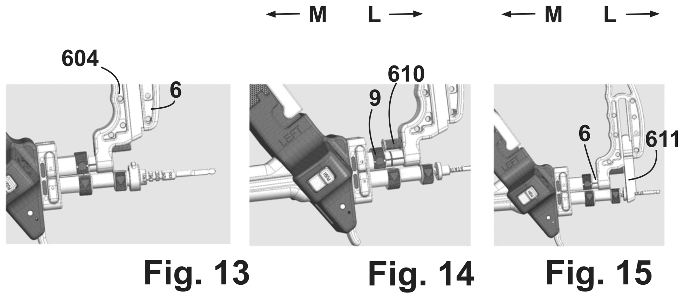

Die mit der Justierschraube 610 sowie der Arretierungseinrichtung 611 erreichten zusätzlichen Funktionalitäten sind anhand von Fig. 13 bis Fig. 15 dargestellt. In Fig. 13 ist eine Situation gezeigt, in welcher eine Knochenschraube 3 bereits in einem Knochenteil wie beispielsweise einem Femurkopf befestigt ist. Die Knochenschraube 3 ist superior angeordnet. Des Weiteren ist das Werkzeug 6 an der Knochenschraube 3 an deren lateralen Ende formschlüssig bzw. umgekehrt aufgenommen. Inferior ist eine Führungshülse ersichtlich, durch welche ein Bohrer geführt ist. Wie ersichtlich ist, kann die Führungshülse trotz der engen Platzverhältnisse lateral noch vorbeigeführt werden, auch wenn das Werkzeug 6 an der Knochenschraube 3 befestigt ist und die Stellschraube 4 samt Führungshülse hierfür leicht schräg zur Längsachse der Knochenschraube 3 und damit auch zur Befestigungsachse B einläuft. Dies ist auch deswegen möglich, weil der Grundkörper 601 in der erläuterten Weise mit einem relativ schmalen distalen Ende 603 samt Aufnahme 609 ausgebildet ist und sich zum ersten Ende 602 hin mit dem Greifbereich 604 verbreitert. Erst wenn die Nut 606 des Werkzeuges 6 aber so ausgerichtet ist, dass die Stellschraube 4 bzw. deren Führungshülse vorbeigeführt werden kann, ist eine korrekte Ausrichtung der Knochenschraube 3 gegeben, wodurch sich die erste Funktionalität ergibt.

Eine zweite Funktionalität wird durch die Justierschraube 610 ermöglicht, wie dies in Fig. 14 ersichtlich ist. Greift das Werkzeug 6 an der Knochenschraube 3 an und liegt das Werkzeug 6 gleichzeitig an einem durch beispielsweise eine Führungshülse für die Stellschraube 4 gebildeten Ende an, bewirkt ein Einschrauben der Justierschraube 610 eine laterale Verschiebung der Knochenschraube 3, sobald die Justierschraube 610 medial beispielsweise, wie in Fig. 14 ersichtlich, an einer Gewebeschutzhülse 9 anstößt. Sofern also superior ein medialer Anschlag gegeben ist (superiore Gewebeschutzhülse), führt ein Einschrauben der Justierschraube 610 bei gegebenem medialen Anschlag zu einer Bewegung der Knochenschraube 3 samt damit verbundenem Knochenteil. Somit kommt es zu einer Annäherung der getrennten Knochenteile, also einer Apposition. Diese Apposition ist begrenzt durch einen Spielraum, welcher von der Stellschraube 4 vorgeben bzw. mit dieser eingestellt wird. Dies ist eine zweite Funktionalität, welche erreicht wird, wenn die Justierschraube 610 vorgesehen ist.

Anhand von Fig. 15 ist eine dritte Funktionalität erläutert. Diese Funktionalität ergibt sich durch die Arretierungseinrichtung 611. Wird die Arretierungseinrichtung 611 betätigt und linear verschoben, also von einer proximalen Ausgangssituation in eine distale Betriebsposition, kann dadurch beispielsweise, wie in Fig. 15 ersichtlich, ein Schraubendreher für die Stellschraube 4 erfasst werden. Da der Schraubendreher für die Stellschraube 4 mit derselben verbunden ist, gleichzeitig aber auch einen Anschlag aufweist, führt die Verbindung über das Werkzeug 6 bei Betätigung des Schraubendrehers entgegen dem Uhrzeigersinn zu einem Herausdrehen der Stellschraube 4 und damit aber auch einer lateralen Bewegung der Knochenschraube 3, wobei diese laterale Bewegung nicht weiter eingeschränkt ist. Somit kann grundsätzlich ein beliebig hoher Druck auf voneinander getrennte Knochenteile in einem Berührungsbereich aufgebracht werden, also eine Kompression. Das Werkzeug 6 ermöglicht somit eine korrekte Positionierung der Knochenschraube 3, wenn diese fehlausgerichtet ist, eine Apposition von Knochenteilen und eine Kompression von Knochenteilen. Dies ist eine dritte Funktionalität, die erreicht wird, wenn die Arretierungseinrichtung 611 vorgesehen ist.

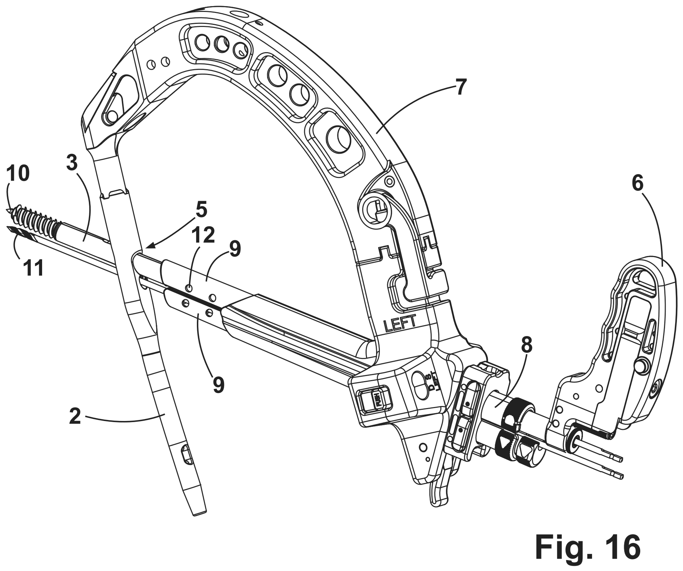

Fig. 16 zeigt das Werkzeug 6 während eines Operationsschrittes. Ein Operationsverfahren kann insbesondere mit folgenden Verfahrensschritten ablaufen: Zunächst wird in einem ersten Schritt ein Knochennagel 2 mit einem Zielgerät 7 in einen Markraum eines Oberschenkelknochens eingebracht. Dabei kann der Knochennagel 2 auch leicht eingehämmert werden. Der Knochennagel 2 weist eine Öffnung 5 auf, welche durchgängig ist und einem proximalen Ende des Knochennagels 2 näher liegt als einem distalen Ende. Die Öffnung 5 bietet Platz zum Aufnehmen und Durchführen einer Knochenschraube 3 sowie einer Stellschraube 4. Das Zielgerät 7 weist hierfür an einem lateral zugewendeten Ende der sichelförmigen Ausbildung ebenfalls eine Öffnung auf, durch welche eine Masterhülse 8 eingeführt werden kann. Ist in dem Oberschenkel eine Inzision eingebracht, können durch die Masterhülse 8 Gewebeschutzhülsen 9 eingebracht werden, welche Sichtöffnungen 12 aufweisen. Die Gewebeschutzhülsen 9 sind mit einem geringen Winkel von etwa 1° bis 1,5° zueinander angestellt.

Sind die Gewebeschutzhülsen 9 eingebracht, kann im Anschluss die operative Versorgung einer proximalen Femurfraktur erfolgen. Hierfür werden zunächst zwei Bohrdrähte 10, **11** eingebracht, wobei in die Gewebeschutzhülsen 9 bzw. die Masterhülse 8 geeignete Führungshülsen für die Bohrdrähte 10, **11** eingeschoben werden. Eine Positionierung der Bohrdrähte 10, **11** kann über die Sichtöffnungen 12 kontrolliert werden. Die Führungshülsen sind dabei so gestaltet, dass der superiore Bohrdraht 10 und der inferiore Bohrdraht **11** parallel zueinander verlaufend eingebracht und anschließend dementsprechend angeordnet sind. Mit den Bohrdrähten 10, **11** wird eine temporäre, interoperative Rotationsstabilität erreicht. Sind die Bohrdrähte 10, **11** nämlich beispielsweise in einen Femurkopf eingebracht, ist keine Rotation des Femurkopfes mehr möglich. Im Anschluss an die Einbringung der Bohrdrähte 10, **11** wird die Führungshülse für den superioren Bohrdraht 10 entfernt und es wird die Knochenschraube 3 eingebracht. Der superiore Bohrdraht 10 kann dann entfernt werden. Die Knochenschraube 3 wird so ausgerichtet, dass eine geradlinige, laterale (in Fig. 15 ersichtliche) Führungslinie am Werkzeug 6 in Übereinstimmung mit einer korrespondierenden Markierung an der superior angeordneten Gewebeschutzhülse 9 fluchtend in Übereinstimmung gebracht wird. Damit sollte die Knochenschraube 3, welche außenseitig eine oder mehrere Nuten 32 zum Eingriff einer Stellschraube 4 aufweist, korrekt positioniert sein. Im Anschluss wird der inferiore Bohrdraht **11** entfernt und es wird eine Führungshülse für einen Bohrer für die Stellschraube 4 eingebracht. Ist die Knochenschraube 3 nicht in idealer Position, beispielsweise weil ein Patient umgelagert wurde oder sich aufgrund anderer Vorgänge die superior angeordnete Gewebeschutzhülse 9 vor der Ausrichtung der Knochenschraube 3 an der Markierung verdreht wurde (wodurch anschließend die Knochenschraube 3 fehlausgerichtet wird), kann dies mit dem Werkzeug 6 durch eine geringfügige Rotation der Knochenschraube 3 korrigiert werden, sodass der Bohrer an der Knochenschraube 3 lateral so weit wie erforderlich vorbeigeführt werden kann. Ein ähnliches Vorgehen ergibt sich, wenn die Stellschraube 4 nicht in ein vorgesehenes, durch eine Nut 32 der Knochenschraube 3 vorgegebenes Berührungsvolumen eingreifen können sollte, sondern in Richtung von lateral nach medial betrachtet bereits zuvor an der Knochenschraube 3 und damit in einer unerwünschten Position anschlägt. Ist die Stellschraube 4 eingeführt, kann im Anschluss gegebenenfalls noch eine Apposition und/oder eine Kompression in der zuvor beschriebenen Weise durchgeführt werden.

## Patentansprüche

1. Werkzeug (6) für ein Set (1) zur Versorgung von Knochenbrüchen, insbesondere proximalen Femurfrakturen, insbesondere ein Set (1) nach einem der Ansprüche 14 bis 16, umfassend einen Grundkörper (601) mit einem ersten, insbesondere proximalen, Ende (602) und einem zweiten, insbesondere distalen, Ende (603) und einem Greifbereich (604) sowie einen Verbindungsbereich (605) zum Anlegen des Werkzeuges (6) an einen Teil des Sets (1), insbesondere eine Knochenschraube (3), **dadurch gekennzeichnet, dass** der Verbindungsbereich (605) eine Nut (606) aufweist.

2. Werkzeug (6) nach Anspruch 1, **dadurch gekennzeichnet, dass** die Nut (606) längserstreckt zwischen einem lateralen Ende (613) und einem medialen Ende (614) verläuft.

3. Werkzeug (6) nach Anspruch 2, **dadurch gekennzeichnet, dass** die Nut (606) relativ zu einer Befestigungsachse (B) schräg verläuft.

4. Werkzeug (6) nach einem der Ansprüche 1 bis 3, **dadurch gekennzeichnet, dass** der Verbindungsbereich (605) schaftförmig ausgebildet ist.

5. Werkzeug (6) nach einem der Ansprüche 1 bis 4, **dadurch gekennzeichnet, dass** der Verbindungsbereich (605) an einem medialen Ende (614) eine Aufnahme zum Aufsetzen und Befestigen des Werkzeuges (6) an einem insbesondere lateralen Ende einer Knochenschraube (3) aufweist.

6. Werkzeug (6) nach einem der Ansprüche 1 bis 5, **dadurch gekennzeichnet, dass** der Grundkörper (601) eine Justierschraube (610) umfasst, wobei die Justierschraube (610) parallel zur Befestigungsachse (B) verläuft.

7. Werkzeug (6) nach Anspruch 6, **dadurch gekennzeichnet, dass** der Grundkörper (601) ein Innengewinde aufweist, in welches ein Außengewinde der Justierschraube (610) eingreift, wobei die Justierschraube (610) länger ausgebildet ist als eine Distanz (D) zwischen einem lateralen Rand (607) und einem medialen Rand (608) im Bereich der Justierschraube (610).

8. Werkzeug (6) nach einem der Ansprüche 1 bis 7, **dadurch gekennzeichnet, dass** das Werkzeug (6) eine Arretierungseinrichtung (611) zur lösbaren Befestigung des Werkzeuges (6) an einem weiteren Teil des Sets (1) umfasst.

9. Werkzeug (6) nach Anspruch 8, **dadurch gekennzeichnet, dass** die Arretierungseinrichtung (611) im Greifbereich (604) angeordnet ist.

10. Werkzeug (6) nach Anspruch 8 oder 9, **dadurch gekennzeichnet, dass** die Arretierungseinrichtung (611) zwischen erstem Ende (602) und zweitem Ende (603) längsverschiebbar gelagert ist.

11. Werkzeug (6) nach einem der Ansprüche 8 bis 10, **dadurch gekennzeichnet, dass** das Werkzeug (6) einen Freigabe- und Fixiermechanismus (612) für die Arretierungseinrichtung (611) umfasst.

12. Werkzeug (6) nach einem der Ansprüche 8 bis **11, dadurch gekennzeichnet, dass** die Arretierungseinrichtung (611) quer zur Befestigungsachse (B) verschiebbar gelagert ist.

13. Werkzeug (1) nach einem der Ansprüche 1 bis 12, **dadurch gekennzeichnet, dass** der Grundkörper (601) im Wesentlichen eben ausgebildet ist.

14. Set (1) zur Versorgung von Knochenbrüchen, insbesondere proximalen Femurfrakturen, umfassend einen Knochennagel (2), eine Knochenschraube (3) und eine Stellschraube (4), wobei der Knochennagel (2) eine Öffnung (5) aufweist, durch welche die Knochenschraube (3) geführt ist, und wobei die Stellschraube (4) eingerichtet ist, mit der Knochenschraube (3) in Kontakt gebracht zu werden, um zumindest eine Bewegungsmöglichkeit der Knochenschraube (3) festzulegen, **dadurch gekennzeichnet, dass** das Set (1) ein Werkzeug (6) zur insbesondere rotierenden Bewegung der Knochenschraube (3) im befestigten Zustand umfasst, insbesondere ein Werkzeug (6) nach einem der Ansprüche 1 bis 13, wobei das Werkzeug (6) eine Nut (606) für die Stellschraube (4) aufweist.

15. Set (1) nach Anspruch 14, **dadurch gekennzeichnet, dass** die Stellschraube (4) in einem Winkel schräg zur Knochenschraube (3) verlaufend im Knochennagel (2) fixierbar ist.

16. Set (1) nach Anspruch 14 oder 15, **dadurch gekennzeichnet, dass** die Stellschraube (4) eingerichtet ist, in ein durch die Knochenschraube (3) definiertes Berührungsvolumen einzugreifen.
